# FASCICULE DE BREVET EUROPEEN

(11) **EP 0 827 727 B1**
(45) Date de publication et mention de la délivrance du brevet: **17.07.2002**
(21) Numéro de dépôt: 97490028.4
(22) Date de dépôt: 28.08.1997
(51) Int. Cl.: A61F 13/15

(54) **Change pour incontinent**
Inkontinenzschutz
Incontinence guard

(30) Priorité: 03.09.1996 FR 9610934
(43) Date de publication de la demande: 11.03.1998
(73) Titulaire: INBRAND FRANCE Société Anonyme, 59290 Wasquehal (FR)
(72) Inventeur: Lahousse, Thierry, 59290 Wasquehal (FR); Vanhecke, David, 59290 Wasquehal (FR); Galand, Nicolas, 59290 Wasquehal (FR)
(74) Mandataire: Duthoit, Michel

(56) Documents cités:
- EP-A- 0 255 500
- EP-A- 0 362 094
- WO-A-95/14453
- US-A- 3 400 718
- US-A- 5 171 236

## Description

La présente invention concerne un change pour incontinent, destiné à être utilisé dans le domaine médical, mais non exclusivement.

Dans ce domaine particulier, il s'est avéré au cours de ces dernières années que les langes traditionnels étaient de plus en plus remplacés par des couches jetables après usage, du type largement diffusé pour les bébés.

Un problème est apparu concernant ce type nouveau de couche jetable pour incontinent, se situant principalement au niveau du maintien en place de ladite couche.

Ce type de problème a été résolu de différentes manières par exemple, en proposant des moyens de maintien formés par au moins un élément de ceinture et fixés dans le sens longitudinal de la couche et qui, en action, se replient transversalement à la couche pour ceinturer l'incontinent.

D'autres moyens de maintien sont connus par ailleurs et consiste à disposer un moyen d'élastification de la ceinture périphérique de manière à enserrer étroitement la taille du patient.

Ce dispositif présente l'inconvénient majeur d'être d'un prix de revient très élevé, du fait même de l'élastification et du procédé de mise en place de tels moyens.

Il a été donc par conséquent imaginé de disposer des moyens d'élastification, en demi-ceinture disposée à l'arrière du change, c'est-à-dire venant en contact avec le dos du patient. Un tel agencement résout partiellement le problème du coût des moyens d'élastification.

Un autre problème est apparu au personnel soignant devant mettre en place de tels changes sur des incontinents. En effet, il a pu être observé que les liquides à absorber, et à retenir grâce au change mis en place, migraient vers la partie postérieure du patient.

C'est ainsi qu'ont été développés des changes avec un matelas absorbant de plus grande dimension vers l'arrière afin d'augmenter son pouvoir absorbé localement.

Cependant, cela a pour inconvénient de rigidifier la partie arrière du change, et de rendre superfétatoire les moyens d'élastification prévus en demi-ceinture arrière.

Par ailleurs, il est connu, dans les changes pour bébé, des bandes élastifiées totales en demi-ceinture avant de manière à permettre un ajustement de la couche, le tour de taille de l'enfant étant susceptible de varier sensiblement, notamment entre deux repas et au fur et à mesure de la digestion.

Pour des adultes, le problème est différent et la difficulté à résoudre porte entre autres sur le bâillement du change après mise en place, compte tenu de ses grandes dimensions.

On connaît également du document EP-A-0.255.500 un change dans lequel une bande élastifiée, située au niveau de la demi-ceinture arrière, est constituée d'un matériau étanche au liquide. Elle s'étend sur toute la longueur de ladite demi-ceinture afin de définir, en combinaison avec la feuille extérieure imperméable du change, une poche étanche où est logée l'extrémité arrière du matelas absorbant.

Une telle structure permet de ne plus avoir à rabattre une partie de la feuille extérieure du change, comme cela était nécessaire auparavant pour ce type de change, mais ne résout en rien les problèmes évoqués plus haut concernant les changes pour incontinents.

Le but de la présente invention est de proposer un change pour incontinent qui pallie les inconvénients précités et favorise l'efficacité du change par une bonne adaptation au porteur.

Par conséquent, les objectifs de la présente invention sont les suivants :
- disposer d'une grande surface absorbante à l'arrière du patient, supérieure à celle disposée à l'avant,
- éviter le bâillement du change au niveau de ladite ceinture,
- réduire au maximum la surface des moyens d'élastification de la ceinture,
- permettre une mise en place symétrique du change sur le patient.

La présente invention permet d'atteindre ces différents objectifs et concerne à cet effet, un change pour incontinent qui trouvera notamment son application dans le domaine médical, composé au moins de manière connue d'une feuille extérieure en matériau imperméable au liquide, d'un matelas d'un matériau absorbant, d'une feuille intérieure en matériau perméable dite de confort, de moyens d'élastification d'une ceinture formée en deux parties, par les zones d'extrémités avant et arrière d'un développé du change, en vue de la mise à la taille ultérieure de l'incontinent, lesdites zones d'extrémité avant et arrière étant situées au-delà dudit matelas, et de moyens de liaison et de maintien des parties avant et arrière de la ceinture, une fois le change en place.

En outre, selon l'invention, ledit matelas est décalé vers l'arrière et lesdits moyens d'élastification sont disposés exclusivement et partiellement sur ladite zone d'extrémité avant.

Plus précisément, le matelas absorbant pourra être disposé asymétriquement entre les feuilles extérieure et intérieure, par rapport à un axe médian transversal, le décalage s'effectuant vers la zone d'extrémité arrière du développé dudit change, de manière à obtenir une protection postérieure de l'incontinent, selon une hauteur supérieure à celle assurant sa protection antérieure, pour une longueur relativement inchangée dudit matelas et dudit développé et, un positionnement de l'incontinent également inchangé.

La présente invention concerne également les caractéristiques qui ressortiront au cours de la description qui va suivre et qui devront être considérées isolément ou selon toutes leurs combinaisons techniques possibles.

Cette description, donnée à titre d'exemple non limitatif, fera mieux comprendre comment l'invention peut être réalisée, en référence aux dessins annexés sur lesquels :
- la figure 1 est une vue en plan d'un développé d'un change selon l'invention,
- les figures 2a, 2b, 2c représentent respectivement, de manière schématique, un change traditionnel selon l'art antérieur mis en place de manière également traditionnelle, un change traditionnel mis en place en favorisant l'arrière du patient, et un change selon l'invention favorisant également l'arrière du patient mais pouvant être mis en place de manière symétrique sur celui-ci.

Le change, désigné globalement sur les figures par le repère 1, comprend de manière connue, au moins :
- une feuille extérieure 2 en matériau imperméable au liquide,
- un matelas 3 d'un matériau absorbant,
- une feuille intérieure 4 en matériau perméable dite de confort,
- des moyens d'élastification 5 d'une ceinture formée en deux parties, par les zones d'extrémités avant Av et arrière Ar d'un développé du change, en vue de la mise à la taille ultérieure de l'incontinent,
- des moyens de liaison et de maintien 6, 7 des parties avant et arrière de la ceinture, une fois le change en place.

Ces moyens de liaison et de maintien 6, 7 peuvent être constitués de manière connue, par des adhésifs ou tous autres moyens tels que notamment des moyens auto-agrippants, connus sous la marque « Velcro ».

En ce qui concerne la nature du matériau imperméable constituant la feuille extérieure 2, celui-ci est notamment un polyéthylène. En ce qui concerne la nature du matériau perméable de la feuille intérieure 4, il s'agira, par exemple, d'un matériau non tissé.

Lesdites feuilles extérieure 2 et intérieure 4 sont, par exemple, préformées de manière à présenter, au niveau desdites zones avant Av et arrière Ar du change, une découpe définissant ladite ceinture.

Quant aux moyens de liaison et de maintien 6, 7, ils sont prévus, notamment, au niveau de ladite découpe.

Selon l'invention, les moyens d'élastification 5 sont disposés exclusivement sur la partie avant de la ceinture. De plus, ils ne couvrent celle-ci que partiellement de manière, notamment, à en réduire son prix de revient.

Il est également à noter que ledit matelas 3 est décalé vers l'arrière afin d'améliorer les capacités d'absorption du change dans cette zone.

Avantageusement, les moyens d'élastification 5 sont disposés sensiblement dans la zone centrale de cette partie avant de ceinture.

Ils peuvent être constitués par une portion de bande de matière élastique, rapportée par tout moyen sur une zone correspondante de ladite partie avant, entre les feuilles extérieure (2) et intérieure (4), comme par exemple au moyen d'adhésif.

On notera également que la bande de matière élastique pourrait être remplacée par une bande de mousse ou de tout autre matériau élastiquement déformable.

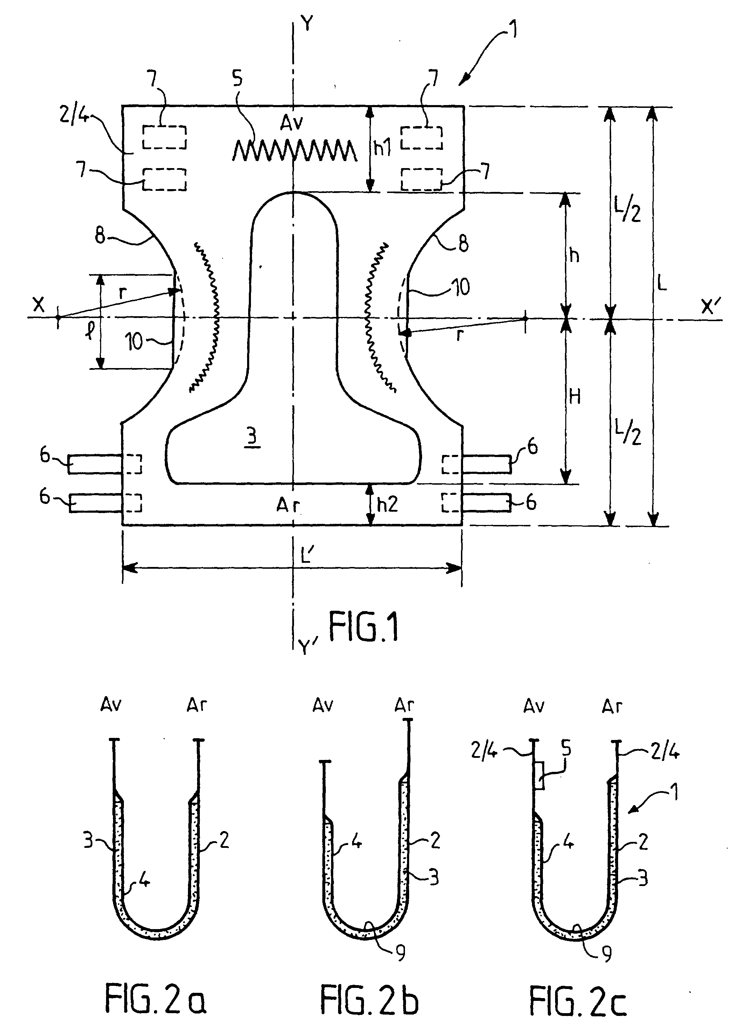

Comme le montrent particulièrement bien les figures 1 et 2c, selon la présente invention le matelas absorbant 3 est disposé asymétriquement entre les feuilles extérieure 2 et intérieure 4, par rapport à un axe médian transversal XX'. Le décalage est effectué vers la zone d'extrémité arrière Ar du développé du change 1.

De cette manière, il est obtenu une protection postérieure de l'incontinent selon une hauteur « H », par rapport à l'axe transversal XX', correspondant à son entrejambe, supérieure à la hauteur « h » assurant sa protection antérieure.

Ceci est obtenu selon une caractéristique avantageuse de l'invention à partir d'une longueur totale de matelas absorbant 3 égale à « H + h », relativement inchangée, par rapport aux changes connus. Egalement grâce à l'invention, le développé de l'ensemble du change 1 et le positionnement sur l'incontinent sont également inchangés, c'est-à-dire qu'avec un change d'un développé identique à celui d'un change traditionnel, il pourra être mis en place sur l'incontinent de manière symétrique, pour son plus grand confort, tout en assurant une absorption maximum dans les zones que l'on a voulu privilégier, c'est-à-dire l'arrière.

Une telle disposition asymétrique du matelas absorbant 3 provoque le décalage vers l'arrière de celui-ci, de longueur « H + h » relativement inchangée, par rapport à la longueur « L » du développé de l'ensemble, libérant ainsi des zones d'extrémités avant Av et arrière Ar formant les deux parties de la ceinture, respectivement plus hautes à l'avant « h₁ » qu'à l'arrière « h ₂ », ladite zone avant Av de hauteur « h1 » supportant les moyens d'élastification 5.

Ceci a pour avantage majeur que le bâillement de la partie avant de la ceinture, de tenue amoindrie par l'importance de sa hauteur « h1 », soit empêché par la présence des moyens d'élastification 5 dans cette zone. En outre, il est à noter une efficacité plus importante des moyens d'élastification car ils sont prévus dans une zone dont la souplesse est accrue notamment du fait de l'absence du matelas absorbant plus rigide.

Selon une autre caractéristique de l'invention, le développé du change 1 comprend deux zones centrales latérales 8 échancrées selon un rayon « r » prédéterminé et délimitant entre elles un entrejambe 9. Ces zones échancrées 8 sont destinées à s'adapter aux cuisses de l'incontinent, le fond desdites échancrures 8 présentant des parties droites ou quasi droites 10 situées en vis-à-vis, sensiblement parallèles à l'axe longitudinal XX' de l'ensemble, et aptes à autoriser selon une plage donnée correspondant à la longueur « I » de cette partie, vers l'avant ou vers l'arrière, selon l'incontinent. Ceci permet notamment d'adapter aux différentes morphologies.

Cette disposition a pour avantage d'éviter tout risque d'irritation des cuisses dans lesdites zones centrales des échancrures 8. A titre d'exemple pratique de réalisation, pour un change d'une longueur totale « L » comprise entre 800 et 1000 mm, la hauteur de la partie « h1 » avant de la ceinture sera comprise entre 175 et 240 mm, alors que la partie « h2 » de la partie arrière Ar de ladite ceinture sera quant à elle comprise entre 50 et 100 mm.

En ce qui concerne les échancrures latérales centrales 8, le rayon « r » de celles-ci sera compris entre 300 et 800 mm, alors que les parties droites ou quasi droites 10 réalisées au fond desdites échancrures 8 auront une longueur comprise entre 100 et 200 mm.

En ce qui concerne la largeur « L' » du développé du change, celle-ci sera comprise entre 400 et 850 mm.

## Revendications

1. Change pour incontinent qui trouvera notamment son application dans le domaine médical, composé au moins d'une feuille extérieure (2) en matériau imperméable au liquide, d'un matelas (3) d'un matériau absorbant, d'une feuille intérieure (4) en matériau perméable dite de confort, de moyens d'élastification (5) d'une ceinture formée en deux parties par les zones d'extrémités avant (Av) et arrière (Ar) d'un développé du change, en vue de la mise à la taille ultérieure de l'incontinent, lesdites zones d'extrémité avant (Av) et arrière (Ar) étant situées au-delà dudit matelas (3), et de moyens de liaison et de maintien (6, 7) des parties avant (Av) et arrière (Ar) de la ceinture une fois le change en place, **caractérisé par le fait que** ledit matelas (3) est décalé vers l'arrière et lesdits moyens d'élastification (5) sont disposés exclusivement sur ladite zone d'extrémité avant (Av).

2. Change selon la revendication 1 dans lequel les moyens d'élastification (5) sont disposés partiellement sur la partie avant de la ceinture.

3. Change selon la revendication 2 dans lequel les moyens d'élastification (5) sont disposés sensiblement dans la zone centrale de la partie avant (Av) de la ceinture.

4. Change selon l'une des revendications 1 à 3 dans lequel les moyens d'élastification (5) sont constitués par une portion de bande de matière élastique rapportée sur une zone correspondante de la partie avant (Av) de la ceinture, entre les feuilles extérieure (2) et intérieure (4).

5. Change selon l'une des revendications 1 à 4 dans lequel le matelas absorbant (3) est disposé asymétriquement entre les feuilles extérieure (2) et intérieure (4), par rapport à un axe médian transversal XX', le décalage s'effectuant vers la zone d'extrémité arrière (Ar) du développé dudit change, de manière à obtenir une protection postérieure de l'incontinent selon une hauteur « H » supérieure à celle « h » assurant sa protection antérieure, pour une longueur totale « H + h » relativement inchangée dudit matelas (3) et dudit développé.

6. Change selon la revendication 5 dans lequel le décalage vers l'arrière du matelas absorbant (3) de longueur « H + h » par rapport à celle « L » du développé de l'ensemble, libère des zones d'extrémités formant les deux parties de la ceinture (Av, Ar) respectivement plus hautes à l'avant « h1 » qu'à l'arrière « h2 », la zone avant (Av) supportant les moyens d'élastification 5.

7. Change selon l'une des revendications 1 à 6 dans lequel son développé comprend deux zones centrales latérales (8), échancrées selon un rayon « r » prédéterminé délimitant entre elles un entrejambe (9) est destiné à s'adapter aux cuisses de l'incontinent, le fond desdites échancrures (8) présentant des parties (10) en vis-à-vis, sensiblement parallèles à l'axe longitudinal YY' de l'ensemble et aptes à autoriser, selon une plage donnée correspondant à la longueur « l » de cette partie, un décalage du change préférentiellement vers l'avant ou vers l'arrière pour l'incontinent.

8. Change selon la revendication 7 dans lequel le rayon « r » des échancrures latérales centrales (8) est compris entre 300 et 800 mm.

9. Change selon l'une des revendications 7 ou 8 dans lequel les parties droites ou quasi droites (10) réalisées au fond des échancrures latérales (8) ont une longueur comprise entre 100 et 200 mm.

10. Change selon l'une des revendications précédentes dans lequel les hauteurs « h1, h2 » des parties avant (Av) et arrière (Ar) de la ceinture sont comprises respectivement entre 175 et 240 mm et entre 50 et 100 mm.

## Patentansprüche

1. Inkontinenzschutzbinde, die nämlich im medizinischen Bereich Anwendung finden wird, bestehend aus wenigstens einer äußeren Folie (2) aus flüssigkeitundurchläßigem Material, einer Einlage (3) aus saugfähigem Material, einer Bequemlichkeitsfolie genannten, inneren Folie (4) aus durchläßigem Material, Mitteln zur Elastifizierung (5) eines durch den vorderen (Av) und den hinteren (Ar) Endbereich einer entfalteten Fläche gebildeten, zweiteiligen Bandes, zwecks der späteren Anpassung an den Taillenumfang des an Inkontinenz Leidenden, wobei sich die genannten vorderen (Av) und hinteren (Ar) Endbereiche jenseits der genannten Einlage (3) befinden, und aus Mitteln zum Verbinden und Halten (6, 7) der vorderen (Av) und hinteren (Ar) Bereiche des Bandes, nachdem die Schutzbinde angebracht worden ist, **dadurch gekennzeichnet, daß** die genannte Einlage (3) nach hinten verlagert ist und die genannten Elastifizierungsmittel (5) ausschließlich an dem genannten vorderen Endbereich (Av) angeordnet sind.

2. Schutzbinde nach Anspruch 1, bei der die Elastifizierungsmittel (5) teilweise am vorderen Teil des Bandes angeordnet sind.

3. Schutzbinde nach Anspruch 2, bei der die Elastifizierungsmittel (5) im wesentlichen im Mittelbereich des vorderen Teils (Av) des Bandes angeordnet sind.

4. Schutzbinde nach einem der Ansprüche 1 bis 3, bei der die Elastifizierungsmittel (5) aus einer Bandlänge aus elastischem Material bestehen, die auf einem entsprechenden Bereich des vorderen Teils (Av) des Bandes, zwischen der äußeren (2) und der inneren (4) Folie angebracht ist.

5. Schutzbinde nach Anspruch 1, bei der die saugfähige Einlage (3) bezüglich einer Quermittelachse XX' asymmetrisch zwischen der äußeren (2) und der inneren (4) Folie angeordnet ist, wobei die Verlagerung zum hinteren Endbereich (Ar) der entfalteten Fläche der genannten Schutzbinde hin erfolgt, derart, daß bei einer verhältnismäßig ungeänderten Gesamtlänge « H + h » der genannten Einlage (3) und der genannten entfalteten Fläche ein hinterer Schutz des an Inkontinenz Leidenden über eine Höhe « H » erreicht wird, die größer ist als diejenige « h », die den vorderen Schutz sichert.

6. Schutzbinde nach Anspruch 5, bei der die Verlagerung nach hinten der saugfähigen Einlage (3) einer Länge «H + h» bezüglich derjenigen «L» der entfalteten Fläche des Ganzen die beiden Teile des Bandes (Av, Ar) bildende Endbereiche freiläßt, die vorne "h1" respektive größer sind als hinten « h2 », wobei der vordere Bereich (Av) die Elastifizierungsmittel 5 trägt.

7. Schutzbinde nach einem der Ansprüche 1 bis 6, bei der die entfaltete Fläche zwei seitliche Mittelbereiche (8) umfaßt, die gemäß einem vorbestimmten Radius « r » ausgeschnitten sind und zwischen ihnen einen Schritt (9) bestimmen, um sich den Oberschenkeln des an Inkontinenz Leidenden anzupassen, wobei der Boden der genannten Ausschnitten (8) im wesentlichen parallel zur Längsachse YY' des Ganzen verlaufende, gegenüberliegende Teile (10) aufweist, geeignet, eine Verlagerung der Schutzbinde, vorzugsweise nach vorne oder nach hinter für den an Inkontinenz Leidenden, gemäß einem der Länge «1» dieses Teils entsprechenden Maßes zu gestatten.

8. Schutzbinde nach Anspruch 7, bei der der Radius « r » der mittleren Seitenausschnitte (8) zwischen 300 und 800 mm liegt.

9. Schutzbinde nach einem der Ansprüche 7 oder 8, bei der die am Boden der Seitenausschnitte (8) vorgesehenen, geraden oder fast geraden Teile (10) eine Länge zwischen 100 und 200 mm haben.

10. Schutzbinde nach einem der vorgehenden Ansprüche 6, bei der die Höhen «h1, h2» des vorderen (Av) und des hinteren (Ar) Teils des Bandes jeweils zwischen 175 und 240 mm und zwischen 50 und 100 mm liegen.

## Claims

1. Briefs for incontinent person which will find an application, in particular, in the medical field, composed of at least an outer sheet (2) made of a material impermeable to liquid, of a pad (3) made of an absorbent material, of an inner sheet (4) made of permeable, so-called comfort, material, of means (5) of elastication of a belt formed in two parts by the front (Av) and rear (Ar) end zones of an extension of the briefs, with a view to subsequent fitting around the waist of the incontinent person, said front (Av) and rear (Ar) end zones being located beyond said pad (3), and of means (6, 7) for connecting and holding the front (Av) and rear (Ar) parts of the belt once the briefs are in place, **characterised by** the fact that said pad (3) is offset towards the rear and said elastication means (5) are disposed exclusively on said front (Av) end zone.

2. Briefs according to claim 1, in which the elastication means (5) are disposed partially on the front part of the belt.

3. Briefs according to claim 2, in which the elastication means (5) are disposed substantially in the central zone of the front (Av) part of the belt.

4. Briefs according to one of claims 1 to 3, in which the elastication means (5) are constituted by a portion of elastic material strip fixed on a corresponding zone of the front (Av) part of the belt, between the outer (2) and inner (4) sheets.

5. Briefs according to one of claims 1 to 4, in which the absorbent pad (3) is disposed asymmetrically between the outer (2) and inner (4) sheets, in relation to a transverse centre line XX', the offset being towards the rear (Ar) end zone of the extension of said briefs, so as to obtain rear protection of the incontinent person to a height "H" greater than that "h" ensuring his or her frontal protection, for a relatively unchanged overall length "H + h" of said pad (3) and of said extension.

6. Briefs according to claim 5, in which the rearward offset of the absorbent pad (3) having a length of "H + h" in relation to the length "L" of the extension of the whole, frees end zones forming the two parts of the belt (Av, Ar) respectively higher at the front "h1" than at the rear "h2", with the front (Av) zone supporting the elastication means (5).

7. Briefs according to one of claims 1 to 6, in which its extension includes two lateral central zones (8), cut out to a predetermined radius "r" delimiting between them a crotch (9) which is designed to fit to the thighs of the incontinent person, with the bottom of said cut-out portions (8) having facing parts (10) substantially parallel to the longitudinal axis YY' of the whole and capable, over a given range corresponding to the length "I" of this part, of allowing the briefs to be shifted preferentially forwards or backwards for the incontinent person.

8. Briefs according to claim 7, in which the radius "r" of the central lateral cut-out portions (8) is between 300 and 800 mm.

9. Briefs according to one of claims 7 or 8, in which the straight, or practically straight, parts (10) provided at the bottom of the lateral cut-out portions (8) are of a length of between 100 and 200 mm.

10. Briefs according to one of the preceding claims, in which the heights "h1, h2" of the front (Av) and rear (Ar) parts of the belt are respectively between 175 and 240 mm and between 50 and 100 mm.
